(19) Europäisches Patentamt
European Patent Office
Office européen des brevets

(11) **EP 2 249 799 B1**

(12) **EUROPEAN PATENT SPECIFICATION**

(45) Date of publication and mention
of the grant of the patent:
**13.03.2013 Bulletin 2013/11**

(21) Application number: **09706636.9**

(22) Date of filing: **16.01.2009**

(51) Int Cl.:
*A61K 9/00* *(2006.01)*  *A61K 47/10* *(2006.01)*
*A61K 47/36* *(2006.01)*  *A61K 47/38* *(2006.01)*

(86) International application number:
**PCT/GB2009/000116**

(87) International publication number:
**WO 2009/095639 (06.08.2009 Gazette 2009/32)**

(54) **FREEZABLE LUBRICANT**

FRIERFÄHIGES SCHMIERMITTEL

LUBRIFIANT POUVANT ÊTRE CONGELÉ

(84) Designated Contracting States:
**AT BE BG CH CY CZ DE DK EE ES FI FR GR HR
HU IE IS IT LI LT LU LV MC MK MT NL NO PL PT
RO SE SI SK TR**

(30) Priority: **29.01.2008 GB 0801601**

(43) Date of publication of application:
**17.11.2010 Bulletin 2010/46**

(73) Proprietor: **LRC Products Limited
Slough, Berkshire SL1 3UH (GB)**
Designated Contracting States:
**AT BE BG CH CY CZ DE DK EE ES FI FR GR HR
HU IE IS IT LI LT LU LV MC MK MT NL NO PL PT
RO SE SI SK TR**

(72) Inventors:
• **LODGE, Rosie**
**London EC4V 6BW (GB)**
• **JENKINSON, Andrew**
**London EC4V 6BW (GB)**
• **ATTRILL, Julie**
**London EC4V 6BW (GB)**

(74) Representative: **Carlin, Robert George et al
Reckitt Benckiser
Corporate Services Limited
Legal Department - Patents Group
Dansom Lane
Hull
HU8 7DS (GB)**

(56) References cited:
**WO-A-2009/087370      US-A- 5 208 031
US-A1- 2005 181 057**

• **ANONYMOUS: "Lubes for Lovers"[Online] 2006,
pages 1-4, XP002551418 Retrieved from the
Internet: URL:http://www.uofaweb.ualberta.ca/
healthi nfo/pdfs/Lubes.pdf> [retrieved on
2009-10-16]**
• **ANONYMOUS: "510(k) Summary
K051295"[Online] 2006, pages 1-5, XP002551419
Retrieved from the Internet: URL:http:
//www.accessdata.fda.gov/cdrh_doc
s/pdf5/K051295.pdf> [retrieved on 2009-10-16]**

**Description**

The present invention relates generally to lubricants, particularly to personal lubricants that are capable of being frozen and used from the frozen state and to a method of making them.

Personal lubricants are specialised lubricants that are used to reduce friction with body tissues. In particular, personal lubricants may be used to provide lubrication, or slip, during sexual activity. For example, personal lubricants can be used to reduce discomfort and/or to increase pleasure during sexual intercourse. However, there is an apparent stigma associated with personal lubricants: some consumers feel embarrassed about buying personal lubricant products, and others perceive personal lubricants as intended for people suffering from a specific problem, such as vaginal dryness, so they do not consider personal lubricants relevant to themselves.

A wide variety of personal lubricants is currently available. These lubricants generally function by supplying water to a body surface in a gelled or viscous form, by including a water-soluble thickener. In use on a body surface, these systems retain a supply of water on the body surface, and water provides lubrication or slip. One or more humectants may be added to aid water retention, and thereby increase the lubrication provided by the lubricant and/or increase the longevity of the lubrication.

A commonly used thickener in personal lubricants is propylene glycol (a low molecular weight glycol). A commonly used humectant is glycerol. Propylene glycol and glycerol are common components of personal lubricants, and are typically used at levels as high as 10% w/w and above. Indeed, in some warming personal lubricants, they are used in place of water as the carrier phase.

It is preferable that personal lubricants provide good levels of lubricity, and that the lubricity is long-lasting, to avoid the need for replenishing or "topping up" the amount of lubricant during use. In general, personal lubricants are also preferably non-tacky, and do not string during use.

Massage lubricants are another class of lubricant. They also serve to reduce friction with body tissues. Many massage lubricants are not suitable for application to the genital area, and in addition many massage lubricants are oil based which means that they are incompatible with some condom types. Accordingly, in contrast to personal lubricants, they are not intended for, and generally are not suitable for, providing lubrication during sexual intercourse.

Combined personal and massage lubricants are known. These are two-in-one products that combine the characteristics of a personal lubricant and a massage gel, and which are condom compatible. These products are therefore suitable for use both as a personal lubricant and as a massage lubricant.

The use of ice cubes (i.e. frozen water) during sexual play is well known. Application of ice to the skin's surface is thought to activate the branch of the nervous system that is responsible for sexual arousal and erection. Indeed, this is reported by Kevin Strang, PhD, a psychologist at the University of Wisconsin School of Medicine and Public Health (see http://www.cosmopolitan.com/sex-love/sex/5-hot-things-ice). The use of ice during sexual play may therefore heighten arousal and excitement.

Kenzo Parfums SA currently markets perfumed ice cubes (Kenzoki Perfumed Ice Cubes). This product is supplied in liquid form and is intended to be frozen for 24 hours prior to use to form solid ice cubes which can then be applied to the skin, simultaneously giving cooling effects and releasing a perfume. The perfumed ice cubes are stated to be made from water extracted from the rice plant, containing plant extracts and perfume.

Standard ice cubes and the Kenzo perfumed ice cubes consist solely and predominantly, respectively, of water. Therefore, as they melt in use, they have the properties of water, and do not have the properties of a personal lubricant or a massage lubricant. Whilst the ice cubes and perfumed ice cubes discussed above give cooling effects, which may provide desirable sensations during sex play, they do not provide any lubrication.

Freezable lubricant compositions are difficult to formulate, because most of the standard components found in personal lubricants cannot be used, as they have the effect of lowering the freezing point of lubricant compositions to such an extent that they cannot be readily frozen, for example, such that they cannot be frozen in a domestic freezer.

The addition of a solute to a solvent will typically depress the freezing point of the solvent. The extent of depression of the freezing point is dependent on the amount of solute and upon the particular solvent. Freezing point depression is a colligative property, that is, it depends on the number of particles in a given volume of solvent, not on the nature of the particles. Freezing point depression can be expressed by the following equation:

$$\Delta T_f = K_f \times m_b = k_f \times m_{solute} \times i$$

Where:

$\Delta T_f$     = freezing point depression from that of the pure solvent
$K_f$     = cryoscopic constant for the solvent [°C/mole, for water $K_f$ = -1.86 °C/mole]

$m_b$ = molality of the solution

$m_{solute}$ = moles solute per kilogram of solvent

$i$ = dissociation factor [for a non-electrolyte, $i$ = 1, for electrolytes it is the number of ions the electrolyte molecule dissociates into in the solvent]

Depending upon the solute, the freezing point depression is such that the formulations do not form coherent crystalline structures at suitable temperatures - that is, between about 0°C and about -22°C, or the structure formed is soft or easily disintegratable or reformable during application to the skin or when force is otherwise applied to the frozen structure.

While all formulations are ultimately capable of being frozen if the temperature is lowered sufficiently, once the temperature of the frozen product drops below about -22°C it becomes unsuitable for use in the context of sexual play, because the potential for the product to cause cold bums when in prolonged contact with the skin increases significantly at temperatures below about -22°C. Whilst in some situations, for example in the removal of warts and verrucas, cold burning is desirable to kill body tissue, cold burning is clearly undesirable in the context of sexual play.

An example of a lubricant which has now been found to be freezable but which does not function as a lubricant in the frozen state, is Sliquid(R) H2O Intimate Lubricant (available from Sliquid LLC). This is a personal lubricant only; it is not intended for use as a massage lubricant. In addition, it is not intended to be frozen: it is not marketed as a freezable lubricant. The ingredients of the Sliquid® lubricant include water, sorbitol, plant cellulose, polyquat 7 and DMDM hydantoin. We have found that this lubricant does not function well as a personal lubricant as it dries quickly and does not have a sufficiently high viscosity. Accordingly, whilst it can be frozen, it does not provide acceptable lubrication. US2005/181057 discloses personal lubricants suitable for lubricating body tissues. US5208031 discloses a sexual lubricant comprising water, a thickening agent and a lubricating agent.

"Lubes for Lovers", [Online] 2006, pages 1-4 [retrieved on 2009-10-16], Retrieved from the Internet: URL: http://www.uofaweb.ualberta.ca/healthinfo/pdfs/Lubes.pdf, discloses a water-based personal lubricant comprising water, humectants and a thickener.

"510(k) Summary K051295", [Online] 2006, pages 1-5, [retrieved on 2009-10-16], Retrieved from the Internet: URL:http://www.accessdata.fda.gov/cdrh_docs/pdf5/K051295.pdf, discloses a water-based personal lubricant comprising water, a humectant, a cellulose polymer and a carbomer thickener.

We have appreciated that there is currently no available product that can be frozen to provide the cooling sensations of ice, and which can also function as a personal lubricant and massage gel during the melting phase, to further enhance the sexual experience. It would be advantageous if such a product could be provided.

According to the present invention in its broadest aspect, there is provided a personal lubricant suitable for adequately lubricating body tissues including skin particularly during sexual activity, which lubricant can be frozen at a temperature of from -22°C to 0°C to form a rigid homogenous solid and which melts during use to provide lubrication, which personal lubricant comprises, water, at least one high molecular weight humectant and slip agent having a molecular weight of from 20 to 35kDa, and at least one high molecular weight thickener and slip agent having a molecular weight of 20kDa, optionally 50kDa, or above, and wherein the lubricant comprises no more than a total of 2% by weight of the lubricant of propylene glycol or glycerol.

The personal lubricant of the present invention can be frozen and provides acceptable lubrication when applied to the skin in the frozen state.

The personal lubricant of the present invention which can be frozen, characterised in that in the frozen state it is a rigid homogenous solid and in the melted state it can have a viscosity of about 5 cP or above and/or an initial coefficient of friction of about 0.05 or below.

Related to the present invention there is also a personal and massage lubricant comprising water; at least one high molecular weight humectant and slip agent; and at least one high molecular weight thickener and slip agent.

By "humectant and slip agent" we mean ingredients that act both as a humectant and as a slip agent. That is, ingredients having the dual functionality of hygroscopicity and slip (or lubricity), when hydrated.

By "thickener and slip agent" we mean ingredients that act both as a thickener and as a slip agent when in solution; many thickeners do not have adequate slip properties in solution, and many slip agents do not have the ability to thicken aqueous solutions. For the purposes of the present invention, however, an ingredient having both these functionalities is required.

Related to the present invention, there is a personal and massage lubricant comprising water; at least one high molecular weight humectant and slip agent selected from high molecular weight polyethylene glycols, poly(ethylene oxide)s, hyaluronic acids and sodium hyaluronate; and at least one high molecular weight thickener and slip agent selected from propylene glycol alginates; cellulose derivatives; alginates; carbomers; carrageenen and xanthan gum.

Related to the present invention, there is a personal and massage lubricant consisting essentially of water; at least one high molecular weight humectant and slip agent; and at least one high molecular weight thickener and slip agent.

Related to the present invention, there is a personal and massage lubricant consisting essentially of water; at least one high molecular weight humectant and slip agent selected from high molecular weight polyethylene glycols, poly(efhylene oxide)s, hyaluronic acids and sodium hyaluronate; and at least one high molecular weight thickener and slip agent selected from propylene glycol alginates; cellulose derivatives; alginates; carbomers; carrageenen and xanthan gum.

Related to the present invention, there is a method for making a personal and massage lubricant comprising water; at least one high molecular weight humectant and slip agent; and at least one high molecular weight thickener and slip agent, comprising the steps of providing water; and adding each of the high molecular weight ingredients to the water separately, ensuring that each ingredient is fully dissolved before addition of the next ingredient.

It is highly preferred that the personal and massage lubricants can be readily frozen in a domestic freezer. Therefore the lubricants have a freezing point of - 22°C or above. A freezing point above -22°C is preferred, as this avoids the risk of cold burns when applied to the skin. The lubricant has a freezing point of between -22°C and O°C. Preferably, the lubricants freeze within 12 hours or less. More preferably, the lubricants freeze within about 1 to 2 hours.

The lubricants can be frozen and can be used as a massage product in the frozen state. As the frozen lubricant melts in use, the lubricant flows onto the skin and provides lubrication. Accordingly, the properties of the lubricant - including viscosity and coefficient of friction - as it melts are important. Preferably, the lubricant has a viscosity in the melting phase of about 5cP or above, more preferably about 7.5 cP or above. It is also preferred that in the melting phase, the lubricant has an initial coefficient of friction of about 0.05 or below, preferably below 0.05, more preferably about 0.02 or below.

It is particularly preferred that, in the melting phase, the lubricant has a viscosity of 5 cP or above and an initial coefficient of friction of 0.05 or below.

We have found that, after freezing, the resultant cubes of frozen lubricant retain their integrity during application and during the melting phase. In particular, we have found that the cubes do not crumble or become soft or mushy in use. This is particularly advantageous because ice cubes that do not retain their integrity during application would be difficult to use, and would not provide the desired sensual benefits.

In the melting phase, the lubricants provide acceptable lubricity. In particular, the lubricants provide a good level of lubricity, and the lubricity is long lasting. In contrast to some of the known lubricants, which are based on natural gum polysaccharides, the lubricants of the invention do not string in use, and do not feel slimy. The personal and massage lubricants of the invention have also been found to be non-tacky.

Another advantage of the lubricants provided herein is that they are stable to repeated freeze-thaw cycles.

Because the lubricants are water-based, they are compatible with condoms. In particular, they are compatible with natural rubber latex condoms, polyurethane condoms and synthetic polyisoprene condoms.

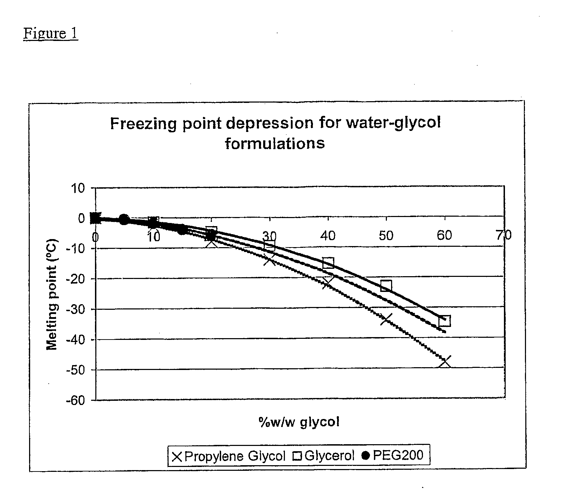

Figure 1 illustrates the effects of propylene glycol and glycerol on freezing point depression of water.

As used herein, the terms "ice cube" and "cube" are used to denote any coherent, three-dimensional piece of ice or frozen personal and massage lubricant, and do not require a cuboidal shape, although a cuboidal shape may be used.

By freezing to form a solid, we mean that the lubricant should freeze so as to give a solid which is capable of being easily removed from the container in which it was frozen. Preferably, the frozen lubricant has physical characteristics similar to those of a pure water ice cube in terms of rigidity and hardness. In particular, it should not be compressible or easily deformed when handled.

By "frozen state", we refer to the personal and massage lubricant after it has been frozen to form a coherent three-dimensional solid.

The term "melting phase" is used to refer to the frozen lubricant after it has been removed from the freezer, as it melts to release liquid lubricant.

The personal and massage lubricants of the invention preferably have a viscosity of 5 cP or above in the melted state, more preferably 7.5 cP or above. It is also preferred that the lubricants have an initial coefficient of friction of about 0.05 or below in the melted state, more preferably below 0.05, still more preferably 0.02 or below. It is particularly preferred that the lubricants have a viscosity of 5cP or above and an initial coefficient of friction of 0.05 or below.

The personal and massage lubricants of the invention can be frozen. The lubricants have a freezing point of about -22°C or above, such that they can be frozen in a domestic freezer. It is preferred that the lubricants can be frozen in a domestic freezer in 12 hours or less, more preferably within 1 to 2 hours. On freezing, the lubricants form a rigid homogenous solid, preferably with similar physical characteristics to a pure water ice cube.

On melting, the personal and massage lubricants provide acceptable lubrication. In particular, in the melted state the lubricant preferably has a viscosity of 5cP or above, more preferably 7.5 cP or above, and/or an initial coefficient of friction of about 0.05 or below in the melted state, more preferably below 0.05, still more preferably 0.02 or below.

It is preferred that the personal and massage lubricants of the invention are freeze-thaw stable, that is, they do not exhibit any significant changes in formulation, structure or performance after a number of freeze-thaw cycles.

Because of freezing point depression, the number of different ingredients should be kept low, so as to minimise depression of the freezing point. However, the lubricants must comprise a certain level of ingredients other than water, in order to function satisfactorily as a lubricant. It is possible to formulate lubricants that consist essentially of water but contain low levels of formulation aids such as thickeners and surfactants. However, although these lubricants freeze satisfactorily, to form frozen cubes of lubricant that retain their integrity, in the melting phase they do not provide acceptable lubrication because they have a low viscosity, and/or are poorly lubricious. We have found that such formulations tend to run off the skin during application, and do not provide sufficient lubricity and feel. Accordingly, lubricants that consist essentially of water and low levels of formulation aids do not provide acceptable lubrication when applied to the skin in the frozen state.

We have found that the majority of the currently available condom-compatible personal lubricants and massage lubricants and gels either cannot be frozen in domestic freezers, because their freezing point is too low, or they can be frozen but do not form coherent ice cubes, instead forming a slush or otherwise easily deformable mass. Examples of lubricants which simply cannot be frozen include oil-based lubricants, for example lubricants based upon silicones, and lubricants including high levels of propylene glycol and/or glycerol.

The majority of water-based personal lubricants contain glycerol (glycerin) and/or propylene glycol and the principal slip agent and/or humectant. However, both propylene glycol and glycerol have a significant effect on freezing point depression. The effect of these ingredients on freezing point is illustrated in Figure 1. It is clear from Figure 1 that both propylene glycol and glycerol act as an antifreeze in water- based formulations, and a similar effect is observed with low molecular weight glycols in general. Accordingly, lubricants based on propylene glycol and/or glycerol will not be capable of freezing to form ice cubes in the required temperature range of -22°C to O°C. In particular, lubricants based on propylene glycol cannot be frozen in the required temperature range, because propylene glycol has a melting point of -59°C. It has now been appreciated that the upper limit for low molecular weight glycols, such as propylene glycol, in lubricants which can be frozen is below about 2% weight, by weight of the total formulation. Accordingly, the lubricants comprise no more than 2% w/w low molecular weight glycols, such as propylene glycol.

Examples of known lubricants which can be frozen but which form a slush or easily deformable frozen mass, rather than forming coherent ice cubes, include most of the leading water based lubricants commercially available in the UK and USA (as determined by sales).

The freezing behaviour of some of these leading personal lubricants was investigated by placing aliquots of lubricant in an ice cube tray in a domestic freezer set at -22°C, and checking the consistency of the lubricant at intervals. The results are summarized below.

| Lubricant | Freezing behaviour (hours at -22°C) | | |
|---|---|---|---|
| | 2 hours | 5 hours | 24 hours |
| Mates Intensify Stimulating Gel[1] (Ansell Ltd) | Thickened slightly, some ice crystals; easily deformed | No change from 2 hours | No change from 2 hours |
| Replens Long-lasting Feminine moisturizer[2] (L'il Drug Store Products Inc) | Many ice particles, crunchy; readily deformed | Finner than at 2 hours | No change from 5 hours |
| Durex Play Feel Lubricant[3] (SSL International plc) | Many ice particles, crunchy; deformable | Firm | No change from 5 hours |
| K-Y Jelly Personal Lubricant[4] (McNeill-PPC Inc) | Few ice particles; readily deformed | No change from 2 hours | No change from 2 hours |
| Durex Play 2-in-1 Massage Gel and Lubricant[5] (SSL International plc) | Few ice particles; readily deformed | Firm | No change from 5 hours |

1 Ingredients are stated to be water, propylene glycol, hydroxyethylcellulose, L-arginine, citric acid, menthol, methylparaben.

2 Ingredients are stated to be water, glycerol, mineral oil, polycarbophil, carbopol 974P, hydrogenated palm oil glyceride, sorbic acid, methylparaben, sodium hydroxide.

3 Ingredients are stated to be water, propylene glycol, hydroxyethylcellulose, benzoic acid, sodium hydroxide.

4 Ingredients are stated as water, glycerol, hydroxyethylcellulose, chlorhexidine gluconate, gluconolactone, methylparaben, sodium hydroxide.

5 Ingredients are stated as water, propylene glycol, hydroxyethylcellulose, PEG 6 caprylic/capric glycerides, benzoic acid, tocopheryl acetate, sodium hydroxide, sodium saccharin.

It is apparent that none of the commercially available lubricants tested freeze to give a coherent solid, even after 24 hours. These commercially available lubricants cannot be frozen. In particular, they cannot be frozen and used as a massage product in the frozen state, because the properties of the lubricant after freezing are not suitable for application to the skin. As can be seen from the details of the ingredients of these commercially available lubricants, they all contain propylene glycol or glycerol (at unknown levels) and, without wishing to be bound by theory, it appears to be the presence of these ingredients that prevents the formulations from freezing.

It has now been found that water-based formulations comprising one or more high molecular weight humectants and slip agents, and low levels of one or more high molecular weight thickeners and slip agents can be successfully frozen, and provide acceptable lubrication when applied to the skin in the frozen state.

In contrast to the commercially available lubricants discussed above, lubricants of the invention have been found to freeze to a non-defonnable solid in a domestic freezer (set at -22°C). Preferably, the lubricants freeze solid within about 12 hours, more preferably within 2 hours.

Any suitable high molecular weight humectant and slip agent can be used. By "humectants and slip agent" we mean ingredients which have a dual function and act both as a humectant and as a slip agent. In the lubricants of the invention, the humectant and slip agent aids in the reduction of water loss from the lubricant during and after the melting phase, as the lubricant is being applied to the skin; the humectant and slip agent also provides the required slip, or lubrication, properties. By high molecular weight, we mean to include humectants and slip agents having a molecular weight of about 20 kDa and above. Humectants and slip agents having a molecular weight of 20kDa or 35kDa are particularly preferred. It has been found that thickeners and slip agents having a high molecular weight, in particular a molecular weight above 20 kDa, have a minimal effect on the freezing point of the lubricant, and provide optimum properties in terms of freezing behaviour and lubricity. The humectant and slip agent may be present in any suitable amount. A preferred amount is from 0.05 to 5% by weight of the total formulation.

Suitable humectants and slip agents include high molecular weight polyethylene glycols; poly(ethylene oxide)s; hyaluronic acids; and sodium hyaluronate. Preferred humectants and slip agents include polyethylene glycols and poly (ethylene oxide)s. Particularly preferred polyethylene glycols have a molecular weight of 20kDa and above, for example a molecular weight of 20 kDa or 35 kDa. For example, preferred polyethylene glycols are commercially available as Polyglykol 20000S and Polyglykol 35000S (Clariant GmbH), having a molecular weight of 20kDa and 35kDa, respectively. Particularly preferred poly(ethylene oxide)s have a molecular weight above 4,000 kDa, for example a molecular weight of 4,400 kDa or 7,200 kDa. Suitable poly(ethylene oxide)s are available under the tradenames RITA PEO-16 (molecular weight 4,400 kDa) and RITA PEO-27 (molecular weight 7,200 kDa) from RITA Corp.

Any suitable thickener and slip agent may be used. By "thickener and slip agent" we mean ingredients which function both as a slip agent and as a thickener when in solution. These dual function ingredients aid the flow properties of the lubricant, particularly during the early melting phase of the frozen lubricant, and also function to enhance the slip, or lubrication, properties. By high molecular weight, we mean in particular thickeners and slip agents having a molecular weight of about 20 kDa and above. Thickeners and slip agents having a molecular weight above 20 kDa are preferred. Thickeners and slip agents having a molecular weight above 50 kD are particularly preferred. The high molecular weight thickener and slip agent is present in the lubricant at a low level. As used herein, "low level" means a level which is low enough to have minimal effects on the freezing point of the lubricant, in particular a level which does not depress the freezing point to below about -22°C. Any suitable amount of thickener and slip agent can be used. A preferred amount is from 0.1 to 5% by weight of the total lubricant formulation. A more preferred amount is from 1.5 to 2.5% w/w.

Suitable thickeners and slip agents include but are not limited to propylene glycol alginates; celluloses, such as hydroxyethylcellulose, hydroxypropylcellulose, hydroxypropylmethylcellulose and carboxymethylcellulose; alginates such as sodium alginate; carbomers; carrageenan; and xanthan gum. Preferred thickeners and slip agents are hydroxyethylcelluloses and propylene glycol alginates. Particularly preferred thickeners and slip agents are hydroxyethylcelluloses available as Natrosol® 250HX, 250M or 250L (Hercules Inc). These hydroxyethylcelluloses have molecular weights of 1000 kDa, 720 kDa and 90 kDa, respectively. A suitable propylene glycol alginate is commercially available as Kelcoloid® S from ISP Alginates, Inc. Kelcoloid® S has a nominal molecular weight of about 40kDa.

The lubricants comprise a high percentage water, by weight of the total lubricant. A suitable amount of water is from about 90% to about 99% w/w.

The lubricants optionally further comprise one or more components selected from pH adjusters; flavours; preservatives and other additives.

A preferred formulation is as follows:

| Ingredient | % w/w |
| --- | --- |
| Humectant and slip agent | 0.05 - 5.00 |
| Thickener and slip agent | 0.10 - 5.00 |
| pH adjuster | To desired pH |

(continued)

| Ingredient | % w/w |
|---|---|
| Flavour | 0.00 - 0.50 |
| Preservative | 0.01 - 0.40 |
| Water | 90 - 99 |

Personal and massage lubricants which can be frozen, according to the present invention, have been found to have several highly advantageous properties. These advantages include freezing to form a hard solid ice cube which is easy to apply; freezing to give ice cubes which stay in place in use, rather than sliding off the skin's surface; freeze-thaw stability; and particularly good lubricant properties in terms of viscosity and lubricity. The lubricants therefore have good freezing and lubricant properties.

These advantageous properties are illustrated by the following tests.

The formulations of the 2-in-1 ice cube lubricants used in the following tests are detailed below:

| Ingredient function | Ingredient | Example | | | | |
|---|---|---|---|---|---|---|
| | | 1 | 2 | 3 | 4 | 5 |
| Water | Water | 97.662 | 97.262 | 95.262 | 94.765 | 97.712 |
| Humectant and slip agent | Polyglykol 35000S[1] | - | 0.500 | 0.500 | 1.000 | - |
| | RITA PEO-18[2] | 0.100 | - | - | - | 0.050 |
| | Propylene glycol | Propylene glycol - | - | 2.000 | 2.000 | - |
| Thickener and slip agent | Natrosol 250HX[3] | - | 1.500 | 1.500 | - | - |
| | Kelcoloid S[4] | 2.000 | 0.500 | 0.500 | 2.000 | 2.000 |
| Preservative | Benzoic acid | 0.150 | 0.150 | 0.150 | 0.150 | 0.150 |
| Flavour (sweetener) | Sodium saccharin | 0.050 | 0.050 | 0.050 | 0.050 | 0.050 |
| pH adjuster | Sodium hydroxide | 0.038 | 0.038 | 0.038 | 0.035 | 0.038 |
| 1 = polyethylene glycol of molecular weight 35kDa, available from Clariant GmbH<br>2 = poly(ethylene oxide) of molecular weight 4,400kDa, available from R.I.T.A Corp.<br>3 = hydroxyethylcellulose of molecular weight 1,000kDa, available from Hercules Inc<br>4 = propylene glycol alginate of molecular weight about 40kDa, available from ISP Alginates Inc | | | | | | |

In order for the personal and massage lubricants of the invention to be freezable and usable as a massage product in the frozen state they should preferably be solid or rigid - in essence, equivalent to a pure water ice cube. The hardness of an ice cube can be measured using a penetration test where a rigid probe is pushed into the ice cube under a fixed load, and the distance penetrated in a fixed time is measured. The deeper the penetration, the softer or less cohesive the ice cube.

The penetration test was carried out using a Stable Micro Systems Ltd (Godalming, UK) Texture Analyser TA-XTplus fitted with a Stable Micro Systems Peltier System 2 Cooling Cabinet to stop the ice cubes melting during testing, and a 30° steel conical probe for the penetration.

The ice and frozen lubricant cubes and probe were placed in the Peltier cabinet, set at -5°C, for 20 minutes before testing. For the test, a cube was placed in a tight-fitting beaker and the conical probe pushed down on the ice cube with a force of 1.5N and the force held at this level for five seconds. The distance the probe penetrated into the cube under this force was then measured. This was repeated five times for each cube, using a fresh position on the cube surface each time, and the mean penetration distance calculated.

The following results were obtained:

| Lubricant | Mean distance penetrated (mm) |
|---|---|
| Control [deionised water] | 1.88 |
| Example 2 | 1.83 |

(continued)

| Lubricant | Mean distance penetrated (mm) |
|---|---|
| Example 3 | 3.68 |
| Example 4 | 5.56 |
| Sliquid® H20 Intimate Lubricant | 1.47 |

It can be seen that Example 2 has a similar hardness to that of pure water. The inclusion of low levels of propylene glycol (2% w/w) in the formulations of Examples 3 and 4, although it did not stop the formulations from freezing, resulted in softer ice cubes which began to crumble very soon after being applied to the skin. In contrast, the currently available Sliquid® formulation resulted in a harder ice cube which felt colder to the touch than the other four ice cubes.

A surprising benefit of the freezable personal and massage lubricant is that the resultant ice cubes were perceived in user tests to be easier to apply and use since they didn't tend to run off the skin during the melting phase. That is, the personal and massage lubricant ice cube stayed in place if left alone, whereas a pure water ice cube (control) and a Sliquid® ice cube didn't.

This behaviour can be determined quantitatively in the following way (slope test):

A 30x10cm aluminium plate (at room temperature) was fixed at an angle of 14° to the horizontal. A 5g ice cube was removed from the ice-tray in the freezer, left at room temperature for about 5 seconds and then placed at the top end of the plate and a stop watch started. The time taken for the cube to slide down the plate a distance of 20cm was then measured. The results are given below:

| Lubricant | Time taken (seconds) |
|---|---|
| Example 2 | >300 |
| Sliquid® H2O | 7-9 |
| Example 5 | 4 |
| Control (deionised water) | 1 |

As can be seen, Example 2 according to the present invention stayed in position on the plate for a considerably longer time than the other ice cubes tested.

In order to determine the stability of the formulation, samples of Example 2 were subjected to twelve freeze-thaw cycles and changes in formulation, structure or performance were observed. Each cycle involved freezing the lubricant for one week at -22° to -25°C, then transferring to a controlled environment room at 25°C, 60% relative humidity for one week, then repeating the cycle. No significant changes in formulation, structure or performance were observed. It can be concluded that the personal and massage lubricant is stable over repeated freeze-thaw cycles.

The lubricant properties of the freezable personal and massage lubricant were assessed, in terms of lubricant viscosity and the coefficiency of friction provided by the lubricant in its melted state.

The coefficient of friction of a lubricant has an inverse relationship to its lubricity; that is, the lower the coefficient of friction the more lubricious the lubricant.

The coefficient of friction of a number of lubricants was measured to compare their relative lubricity. This was carried out using the Micro Systems Texture Analyser TA-XTplus fitted with a Stable Micro Systems Coefficient of Sliding Friction Rig.

The basic test method involves a weighted sled being pulled and pushed back and forth at a fixed speed across a layer of lubricant, and the force required to pull/push the sled measured and averaged over a fixed period of time.

In particular, the lubricant in its melted state was added to the base plate and levelled using a 400$\mu$m bar applicator such that the lubricant spread was spread to all edges of the base plate. The sled was then placed carefully on the lubricant layer, taking care not to press it down onto the lubricant, and the connector to the Texture Analyser screwed in place. A 200g weight was then placed on the sled in the marked area, making a combined weight of 500g. The sled was then moved back and forth across the lubricant layer a distance of 75mm each way, at a speed of 4mm/s for 300 seconds, and the average force calculated. The coefficient of friction was then calculated by dividing the average force required to move the sled, by the vertical force on the lubricant (the mass of the sled plus the added weight).

In addition to the coefficient of friction tests, the viscosity of the various lubricants was measured at low shear rates using a Bohlin CVO Rheometer (available from Malvem Instruments Ltd, Malvern, UK) using cone and plate (CP4"/40mm) geometry. The viscosities measured at a shear rate of 1 s$^{-1}$ at ambient temperature (25°C) are given below, for four commercially available lubricants (K-Y Jelly Personal Lubricant, Durex® Play Feel, Durex Play 2 in 1, and Sliquid® H20 Intimate Lubricant) as well as two lubricant compositions according to the invention (Examples 1 and 2).

The results for the coefficient of friction and viscosity tests are summarised below.

| Lubricant | Coefficient of friction | | Viscosity (cP) measured at a shear rate of $1 \ s^{-1}$ at 25°C |
|---|---|---|---|
| | 0 seconds | 300 seconds | |
| K-Y Jelly Personal Lubricant | 0.85 | 1.00 | 64.0 |
| Durex Play Feel Lubricant | 0.54 | 0.66 | 33.8 |
| Durex Play 2inl Massage Gel and Lubricant | 0.25 | 0.33 | - |
| Sliquid H2O Intimate Lubricant | 0.06 | 0.10 | 1.8 |
| Example 2 | 0.05 | 0.10 | 8.9 |
| Example 1 | 0.02 | 0.07 | - |

The coefficients of friction of two particularly preferred lubricant formulations according to the invention (Examples 1 and 2) are very low, indicating a superior lubricity to that of most of the other lubricants tested. Although Sliquid® H2O has a low coefficient of friction as well, its viscosity is too low, close to that of water [1 cP], so it can be expected to provide no, or poor, lubrication in use.

This coefficient of friction and viscosity data demonstrates that the personal and massage lubricants of the invention have superior slip properties compared to currently available formulations, in addition to the advantageous freezing properties discussed above.

The following Examples illustrate the invention.

Examples 1 and 2

| Ingredient function | Ingredient | % w/w | |
|---|---|---|---|
| | | Example 1 | Example 2 |
| Water | Water | 97.662 | 97.262 |
| Humectant and slip agent | Polyglykol 35000S[1] | - | 0.500 |
| | RITA PEO-18[2] | 0.100 | - |
| Thickener and slip agent | Natrosol 250HX[3] | - | 1.500 |
| | Kelcoloid S[4] | 2.000 | 0.500 |
| Preservative | Benzoic acid | 0.150 | 0.150 |
| Flavour (sweetener) | Sodium saccharin | 0.050 | 0.050 |
| pH adjuster | Sodium hydroxide | 0.038 | 0.038 |
| 1 polyethylene glycol of molecular weight 35 kDa (Clariant GmbH) 2 poly(ethylene oxide) of molecular weight 4,400 kDa (RITA Corp) 3 hydroxyethylcellulose of molecular weight 1,000 kDa (Hercules Inc) 4 propylene glycol alginate (ISP Alginates Inc) | | | |

Example 3

The freezable lubricant of Example 2 is formulated according to the following method.

90% of the water is added to a vessel. The polyethylene glycol is added, with stirring. The benzoic acid is the added to the water with stirring, and the solution mixed until uniform and lump free. The sodium hydroxide and sodium saccharine are mixed with the remaining water and then added to the vessel with stirring. The benzoic acid is thereby neutralised before addition of the propylene glycol alginate. Propylene glycol alginate is then added to the vessel with stirring, and mixed until the batch is clear, uniform and lump-free. Finally, the pH is adjusted to from pH3.8 to pH4.8.

**Claims**

1. A personal lubricant suitable for adequately lubricating body tissues including skin particularly during sexual activity, which lubricant can be frozen at a temperature of from -22°C to 0°C to form a rigid homogenous solid and which melts during use to provide lubrication, which personal lubricant comprises, water, at least one high molecular weight humectant and slip agent having a molecular weight of from 20 to 35kDa, and at least one high molecular weight thickener and slip agent having a molecular weight of 20kDa, optionally 50kDa, or above and wherein the lubricant comprises no more than a total of 2% by weight of the lubricant of propylene glycol or glycerol.

2. A personal lubricant according to claim 1 wherein the lubricant functions as both a personal lubricant and a massage gel.

3. A personal lubricant according to claim 1 or 2 wherein in the melted state it has a viscosity of 5 mPa.s (cP) or above, when measured at a shear rate of 1 s$^{-1}$ at 25°C.

4. A personal lubricant according to claim 1, 2 or 3 wherein in the melted state the lubricant has an initial coefficient of friction of 0.05 or below.

5. A personal lubricant according to claim 1, 2, 3 or 4 wherein the high molecular weight humectant and slip agent comprises one or more of a high molecular weight polyethylene glycol, a poly(ethylene oxide), a hyaluronic acid or a sodium hyaluronate; and the high molecular weight thickener and slip agent comprises one or more of a propylene glycol alginate, a cellulose derivative, an alginate, a carbomer, a carrageenan, or xanthan gum.

6. A personal lubricant according to any one of claims 1 to 5 wherein the high molecular weight humectant and slip agent is present in an amount of from 0.05% to 5% by weight of the total formulation.

7. A personal lubricant according to any one of claims 1 to 6 wherein the high molecular weight humectant and slip agent is present in an amount of from 0.1% to 5% by weight of the total formulation.

8. A personal lubricant according to any preceding claim wherein the lubricant has a freezing point of -22°C or above, or is from -22°C to 0°C.

9. A personal lubricant according to any preceding claim wherein the lubricant freezes to a rigid homogenous solid within 1 to 2 hours or less.

10. A personal lubricant according to any preceding claim wherein in the melted state the lubricant has a viscosity of 7.5 mPa.s (cP) or above, when measured at a shear rate of 1 s$^{-1}$ at 25°C, and an initial coefficient of friction of 0.02 or below.

11. A personal lubricant according to any preceding claim wherein the lubricant is stable to repeated freeze-thaw cycles.

12. A personal lubricant according to any preceding claim wherein the lubricant comprises water in an amount of from 90% to 99% by weight of the lubricant.

13. A personal lubricant according to any one of claims 1 to 12 wherein the high molecular weight humectant and the slip agent comprises a polyalkylene glycol and a poly(alkylene oxide), and the high molecular weight thickener and slip agent comprises a hydroxyl- or carboxyalkylcellulose and an alginate.

14. A two-in-one personal lubricant and massage gel product comprising a lubricant composition as defined in any preceding claim.

**Patentansprüche**

1. Körpergleitmittel, geeignet zum geeigneten Schmieren von Körpergeweben, einschließlich Haut, insbesondere während sexueller Aktivität, welches Gleitmittel bei einer Temperatur von -22 °C bis 0 °C gefroren werden kann, um einen festen, homogenen Feststoff zu bilden, und das während der Verwendung schmilzt, um Schmierung bereitzustellen, welches Körpergleitmittel Wasser, wenigstens ein hochmolekulares Feuchthaltemittel und Schlüpfrigkeitsmittel mit einem Molekulargewicht von 20 bis 35 kDa und wenigstens ein hochmolekulares Verdickungsmittel und Schlüpfrigkeitsmittel mit einem Molekulargewicht von 20 kDa, gegebenenfalls 50 kDa oder darüber, umfasst, wobei das Gleitmittel nicht mehr als insgesamt 2 Gew.-% des Gleitmittels an Propylenglycol oder Glycerol umfasst.

2. Körpergleitmittel gemäß Anspruch 1, wobei das Gleitmittel sowohl als Körpergleitmittel als auch als Massage-Gel wirkt.

3. Körpergleitmittel gemäß Anspruch 1 oder 2, wobei es im geschmolzenen Zustand eine Viskosität von 5 mPa.s (cP) oder darüber, wenn gemessen bei einer Scherrate von 1 s$^{-1}$ bei 25 °C, aufweist.

4. Körpergleitmittel gemäß Anspruch 1, 2 oder 3, wobei das Gleitmittel im geschmolzenen Zustand einen initialen Reibungskoeffizienten von 0,05 oder darunter aufweist.

5. Körpergleitmittel gemäß Anspruch 1, 2, 3 oder 4, wobei das hochmolekulare Feuchthaltemittel und Schlüpfrigkeitsmittel eines oder mehrere von einem hochmolekularen Polyethylenglycol, einem Poly(ethylenoxid), einer Hyaluronsäure und einem Natriumhyaluronat umfasst; und das hochmolekulare Verdickungsmittel und Schlüpfrigkeitsmittel eines oder mehrere von Propylenglycolalginat, einem Cellulosederivat, einem Alginat, einem Carbomer, einem Carrageen und Xanthan umfasst.

6. Körpergleitmittel gemäß einem der Ansprüche 1 bis 5, wobei das hochmolekulare Feuchthaltemittel und Schlüpfrigkeitsmittel in einer Menge von 0,05 Gew.-% bis 5 Gew.-% der Gesamtformulierung vorhanden ist.

7. Körpergleitmittel gemäß einem der Ansprüche 1 bis 6, wobei das hochmolekulare Feuchthaltemittel und Schlüpfrigkeitsmittel in einer Menge von 0,1 Gew.-% bis 5 Gew.-% der Gesamtformulierung vorhanden ist.

8. Körpergleitmittel gemäß einem der vorstehenden Ansprüche, wobei das Gleitmittel einen Gefrierpunkt von -22 °C oder darüber oder von -22 °C bis 0 °C aufweist.

9. Körpergleitmittel gemäß einem der vorstehenden Ansprüche, wobei das Gleitmittel innerhalb von 1 bis 2 Stunden oder weniger zu einem starren, homogenen Feststoff gefriert.

10. Körpergleitmittel gemäß einem der vorstehenden Ansprüche, wobei das Gleitmittel im geschmolzenen Zustand eine Viskosität von 7,5 mPa.s (cP) oder darüber, wenn gemessen bei einer Scherrate von 1 s$^{-1}$ bei 25 °C, und einen initialen Reibungskoeffizienten von 0,02 oder darunter aufweist.

11. Körpergleitmittel gemäß einem der vorstehenden Ansprüche, wobei das Gleitmittel gegenüber wiederholten Gefrier-Auftau-Zyklen stabil ist.

12. Körpergleitmittel gemäß einem der vorstehenden Ansprüche, wobei das Gleitmittel Wasser in einer Menge von 90 Gew.-% bis 99 Gew.-% des Gleitmittels umfasst.

13. Körpergleitmittel gemäß einem der Ansprüche 1 bis 12, wobei das hochmolekulare Feuchthaltemittel und Schlüpfrigkeitsmittel ein Polyalkylenglycol und ein Poly(alkylenoxid) umfasst und das hochmolekulare Verdickungsmittel und Schlüpfrigkeitsmittel eine Hydroxy- oder Carbocyalkylcellulose und ein Alginat umfasst.

14. Zwei-in-einem-Körpergleitmittel- und Massage-Gel-Produkt, umfassend eine Gleitmittelzusammensetzung gemäß einem der vorstehenden Ansprüche.

**Revendications**

1. Lubrifiant intime approprié pour assurer une lubrification adéquate de tissus corporels y compris la peau, en particulier durant des activités sexuelles, ledit lubrifiant pouvant être congelé à une température de -22 °C à 0 °C pour former un solide homogène rigide et fondant en cours d'utilisation pour assurer une lubrification, ledit lubrifiant intime comprenant de l'eau, au moins un humectant et agent de glissement à haute masse moléculaire ayant une masse moléculaire de 20 kDa à 35 kDa, et au moins un épaississant et agent de glissement à haute masse moléculaire ayant une masse moléculaire de 20 kDa, éventuellement de 50 kDa, ou supérieure, et le lubrifiant ne comprenant pas plus de 2 % en poids au total de propylène glycol ou de glycérol relativement au poids du lubrifiant.

2. Lubrifiant intime selon la revendication 1, le lubrifiant pouvant être utilisé comme lubrifiant intime ou comme gel de massage.

3. Lubrifiant intime selon la revendication 1 ou 2, lequel, dans l'état fondu, a une viscosité de 5 mPa.s (cP) ou supérieure, mesurée à une vitesse de cisaillement de 1 s$^{-1}$ à 25 °C.

4. Lubrifiant intime selon la revendication 1, 2 ou 3, le lubrifiant, dans l'état fondu, ayant un coefficient de friction initial de 0,05 ou inférieur.

5. Lubrifiant intime selon la revendication 1, 2, 3 ou 4, dans lequel l'humectant et agent de glissement à haute masse moléculaire comprend un ou plusieurs d'un polyéthylène glycol à haute masse moléculaire, d'un poly(oxyde d'éthylène), d'un acide hyaluronique ou d'un hyaluronate de sodium ; et l'épaississant et agent de glissement à haute masse moléculaire comprend un ou plusieurs d'un alginate de propylène glycol, d'un dérivé de cellulose, d'un alginate, d'un carbomère, d'un carraghénane, ou d'une gomme xanthane.

6. Lubrifiant intime selon l'une quelconque des revendications 1 à 5, dans lequel l'humectant et agent de glissement à haute masse moléculaire est présent dans une quantité représentant 0,05 % à 5 % en poids de la formulation totale.

7. Lubrifiant intime selon l'une quelconque des revendications 1 à 6, dans lequel l'humectant et agent de glissement à haute masse moléculaire est présent dans une quantité représentant 0,1 % à 5 % en poids de la formulation totale.

8. Lubrifiant intime selon l'une quelconque des revendications précédentes, le lubrifiant ayant un point de congélation de -22 °C ou supérieur, ou étant à une température de -22 °C à 0 °C.

9. Lubrifiant intime selon l'une quelconque des revendications précédentes, le lubrifiant se congelant sous la forme d'un solide homogène rigide en 1 à 2 heures ou moins.

10. Lubrifiant intime selon l'une quelconque des revendications précédentes, le lubrifiant, dans l'état fondu, ayant une viscosité de 7,5 mPa.s (cP) ou supérieure, mesurée à une vitesse de cisaillement de 1 s$^{-1}$ à 25 °C, et un coefficient de friction initial de 0,02 ou inférieur.

11. Lubrifiant intime selon l'une quelconque des revendications précédentes, le lubrifiant restant stable quand il est soumis à des cycles de congélation-décongélation répétés.

12. Lubrifiant intime selon l'une quelconque des revendications précédentes, le lubrifiant comprenant de l'eau dans une quantité représentant 90 % à 99 % en poids du lubrifiant.

13. Lubrifiant intime selon l'une quelconque des revendications 1 à 12, dans lequel l'humectant et agent de glissement à haute masse moléculaire comprend un polyalkylène glycol et un poly(oxyde d'alkylène), et l'épaississant et agent de glissement à haute masse moléculaire comprend une hydroxyalkylcellulose ou une carboxyalkylcellulose et un alginate.

14. Produit deux en un lequel est à la fois un lubrifiant intime et un gel de massage, comprenant une composition de lubrifiant selon l'une quelconque des revendications précédentes.

Figure 1

Freezing point depression for water-glycol formulations

**EP 2 249 799 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005181057 A

- US 5208031 A

**Non-patent literature cited in the description**

- *Lubes for Lovers,* 16 October 2009, 1-4

- *510(k) Summary K051295,* 16 October 2009, 1-5